(19) Europäisches Patentamt
European Patent Office
Office européen des brevets

(11) Numéro de publication: **0 112 441**
**A2**

(12) **DEMANDE DE BREVET EUROPEEN**

(21) Numéro de dépôt: **83109216.8**

(22) Date de dépôt: **17.09.83**

(51) Int. Cl.³: **C 07 C 175/00**, C 07 F 7/18, C 07 D 309/12

(30) Priorité: **18.11.82 CH 6713/82**

(43) Date de publication de la demande: **04.07.84 Bulletin 84/27**

(84) Etats contractants désignés: **CH DE FR GB LI NL**

(71) Demandeur: **FIRMENICH SA, 1, route des Jeunes, CH-1211 Geneve 8 (CH)**

(72) Inventeur: **Schulte-Elte, Karl-Heinrich, Dr., 44, chemin de Carabot, CH-1213 Onex (CH)**
Inventeur: **Müller, Bernard Lucien, 16, route de Malagnou, CH-1208 Geneve (CH)**

(74) Mandataire: **Salvadori, Giuseppe, Dr., c/o Firmenich S.A. Case Postale 239, CH-1211 Genève 8 (CH)**

(54) **Carbinols acétyléniques, leur utilisation à titre de produits de départ pour la préparation de bêta-damascénone et carbinols alléniques en tant qu'intermédiaires dans ladite préparation ; procédés pour leur préparation.**

(57) On décrit des composés de formule

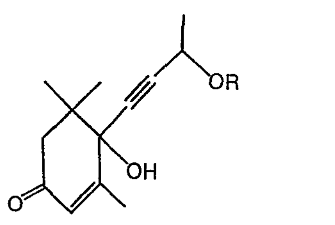

(I)

et de formule

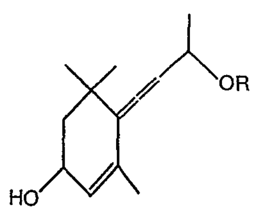

(IV)

dans lesquelles le symbole R désigne un radical trialkylsilyle, un radical alkyle $C_1$–$C_6$, de préférence méthyle, tert-butyle ou isoamyle ou un groupe de formule

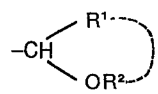

(II)

dans laquelle $R^1$ et $R^2$, pris séparément, représentent chacun un reste alkyle inférieur, de préférence de $C_1$ à $C_3$, ou, pris conjointement, un groupe tétraméthylène.

On décrit également un procédé pour leur préparation ainsi que leur utilisation à titre de produits de départ pour la préparation de β-damascénone.

EP 0 112 441 A2

## Carbinols acétyléniques, leur utilisation à titre de produits de départ pour la préparation de bêta-damascénone et carbinols alléniques en tant qu'intermédiaires dans ladite préparation ; procédés pour leur préparation

La présente invention a pour objet des composés acétyléniques nouveaux, utiles à titre de produits de départ pour la préparation d'une cétone alicyclique, la β-damascénone plus précisément. Lesdits composés sont représentés au moyen de la formule que voici

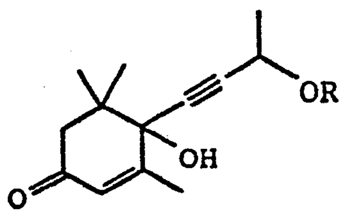

(I)

dans laquelle le symbole R représente un radical trialkyl-silyle, un radical alkyle $C_1$-$C_6$, de préférence méthyle, tert-butyle ou isoamyle ou un groupe de formule

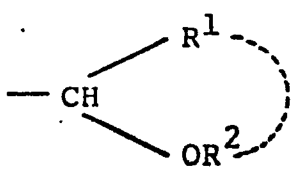

(II)

dans laquelle $R^1$ et $R^2$, pris séparément, représentent chacun un reste alkyle inférieur, de préférence de $C_1$ à $C_3$, ou, pris conjointement, un groupe tétraméthylène.

L'invention a également pour objet un procédé pour la préparation desdits composés de formule (I), caractérisé en ce qu'on fait réagir la 2,2,6-triméthyl-cyclohex-5-ène-1,4-dione (définie ci-après "oxophorone") avec un dérivé organo-métallique de formule

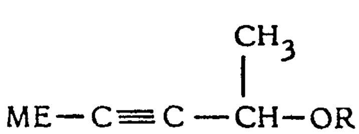

(III)

dans laquelle le symbole ME représente un radical halomagnésien et R représente un radical tel que défini ci-dessus pour la formule (I), et que l'on hydrolyse ensuite le produit de réaction ainsi obtenu.

L'invention a en outre pour objet l'utilisation desdits composés de formule (I) à titre de produits de départ pour la préparation de β-damascénone, caractérisée en ce qu'on soumet sucessivement lesdits composés de formule (I) à une réduction et à un traitement au moyen d'un agent acide.

L'invention a encore pour objet des composés hydroxy-alléniques de formule

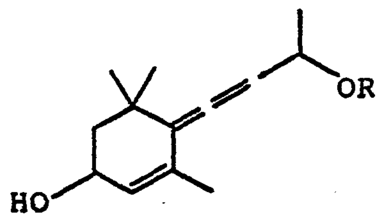

(IV)

dans laquelle R est défini comme indiqué plus haut pour la formule (I), ainsi qu'un procédé pour leur préparation, lequel procédé est caractérisé par la réaction de la 2,2,6-triméthyl-cyclohex-5-en-1,4-dione avec un dérivé organométallique de formule

$$ME-C\equiv C-\overset{\overset{CH_3}{|}}{CH}-OR \qquad (III)$$

dans laquelle les symboles ME et R ont le sens indiqué plus haut, suivie de la réduction du produit de réaction résultant à l'aide d'un hydrure métallique dans un milieu constitué par un solvant organique inerte.

La β-damascénone, une cétone alicyclique de formule

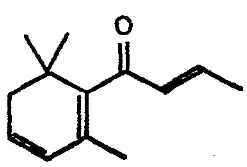

est particulièrement appréciée dans l'art des parfums et arômes pour ses intéressantes propriétés organoleptiques. Son emploi à titre d'ingrédient parfumant, respectivement aromatisant, fait l'objet de nombreux brevets, par exemple US 3 975 310, CH 520 479 et CH 509 399.

Au vu de l'état présent de la technique, la β-damascénone peut être préparée selon divers procédés, présentant plus ou moins d'intérêt du point de vue industriel. Elle s'obtient principalement par

a) déshydrogénation du composé cyclohexénique correspondant, la β-damascone (voir brevet CH 505 773) ;

0112441

b) traitement de la 1-(2,6,6-triméthyl-1,2-époxy-cyclohexyl)-but-2-ène-1-one au moyen d'un agent déshydratant acide (voir DE-AS 2 065 322) ; ou

c) traitement du 4-(2,6,6-triméthyl-1-hydroxy-cyclohex-2-ène-1-yl)-but-3-yne-2-ol au moyen d'un agent déshydratant acide (voir DE-PS 2 242 751).

En dépit des nombreuses synthèses connues à ce jour, il y a malgré tout nécessité soit d'améliorer les procédés existants, soit d'élaborer de nouvelles synthèses, originales, partant de matières premières plus aisément accessibles ou meilleur marché. La présente invention permet de répondre efficacement aux exigences posées ci-dessus.

La première étape des procédés de l'invention consiste à faire réagir la 2,2,6-triméthyl-cylohex-5-ène-1,4-dione avec un dérivé organo-métallique acétylénique de formule (III) telle que définie plus haut, selon les conditions usuelles en chimie organique. On utilise à de telles fins un dérivé organo-métallique de formule (III) dans laquelle le symbole ME représente un radical halogéno-magnésien tel MgBr, MgCl ou MgI. On utilise de préférence le dérivé bromo-magnésien correspondant, dans les conditions d'une réaction dite de Grignard.

Comme indiqué plus haut, le symbole R peut représenter un radical trialkyl-silyle, tert-butyle ou un groupe de formule

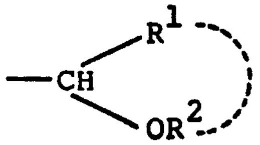

(II)

dans laquelle les symboles $R^1$ et $R^2$, identiques ou différents, pris séparément, représentent chacun un reste alkyle inférieur, de préférence de $C_1$ à $C_3$, par exemple, méthyle, éthyle ou propyle. On peut notamment utiliser un dérivé organo-métallique de formule (III) dans laquelle le symbole R représente le radical 3-oxa-pent-2-yle ($R^1$ = méthyle, $R^2$ = éthyle dans la formule II). On peut tout aussi avantageusement utiliser un dérivé organo-métallique de formule (III) dans laquelle le symbole R représente un radical tétrahydropyranne-2-yle ($R^1 + R^2$ = tétraméthylène dans la formule II). En fait, tout groupe protecteur de la fonction hydroxyle peut être valablement utilisé à cet effet, à condition qu'il soit stable en milieu alcalin et aisément scindable en milieu acide.

Le produit résultant de la réaction d'addition décrite ci-dessus peut, selon une variante d'un procédé de l'invention, être directement réduit à l'aide d'un hydrure métallique, par exemple au moyen de sodium-aluminohydrure diéthylique (OMH), généralement dans un solvant inerte constitué par un hydrocarbure aromatique comme le toluène ou un éther, par exemple l'éther diéthylique ou le tétrahydrofuranne. Une telle procédure permet l'obtention d'un composé allénique de formule

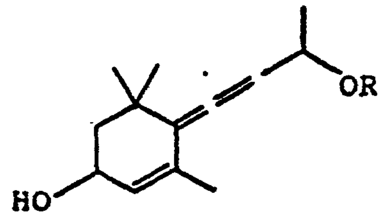

(IV)

lequel, après traitement au moyen d'un agent acide, donne la β-damascénone désirée.

Si l'on désire par contre isoler les composés de formule (I), on hydrolyse selon les techniques usuelles le composé résultant de l'addition de Grignard. La réduction suivante de ce dernier pour fournir le composé (IV) peut être effectuée à l'aide d'un hydrure métallique, selon des conditions de réactions analogues à celles mentionnées plus haut pour la réduction directe. C'est ainsi qu'on peut effectuer une telle réduction au moyen d'OMH, d'aluminohydrure de lithium ou encore de DiBAH (aluminohydrure diisobutylique).

Afin d'obtenir la β-damascénone désirée, le carbinol allénique (IV) résultant est ensuite soumis à un traitement au moyen d'un agent acide. Ledit traitement s'effectue à l'aide d'un acide fort dilué, minéral ou organique, tel l'acide sulfurique, phosphorique, chlorhydrique, acétique, trichloroacétique ou formique par exemple, soit en milieu aqueux, soit en milieu organique anhydre, de préférence l'acide formique dilué.

A titre de solvant organique inerte, on peut utiliser un éther, comme le dioxanne ou le tétrahydrofuranne, ou un hydrocarbure ou mélange d'hydrocarbures. Quoique la température n'ait pas un rôle déterminant, nous avons observé qu'en opérant à une température proche de la température d'ébullition du mélange réactionnel, les rendements étaient les meilleurs et les temps de réaction les plus courts. La β-damascénone ainsi obtenue est finalement isolée et purifiée selon les techniques usuelles, extraction et distillation fractionnée par exemple. Du point de vue organoleptique, elle est tout à fait conforme aux critères de qualité requis par l'industrie des parfums et arômes.

Il convient de relever finalement que les composés de formule (I) définis précédemment sont des composés nouveaux, tout comme par ailleurs les carbinols alléniques (IV).

Les composés organo-métalliques de formule (III) utilisés à titre de produits de départ selon le procédé de l'invention s'obtiennent conformément aux techniques ayant cours en chimie organique, en traitant le dérivé acétylénique correspondant au moyen d'un halogénure d'alkyl-magnésium.

L'oxophorone est un composé connu qui peut, notamment, être obtenu à l'échelle industrielle suivant le procédé décrit dans le brevet US 3 944 620.

L'invention sera illustrée de façon plus détaillée par les exemples ci-après (températures en degrés centigrades).

## Exemple 1

2,6,6-Triméthyl-1-hydroxy-1-(3-tert-butoxy-1-butyne-1-yl)-2-cyclohexène-4-one

### Méthode A

A une suspension de 6,24 g (0,26 atome-gr) de magnésium en copeaux dans 30 ml d'éther anhydre, on a ajouté goutte à goutte une solution de 28,4 g (0,26 Mole) de bromure d'éthyle dans 500 ml d'éther anhydre. Après disparition complète du magnésium, le mélange a été refroidi à température ambiante et on y a ajouté 32,8 g (0,26 Mole) de butynyl-tert-butyle éther dans 50 ml d'éther et le tout a été maintenu sous agitation à température ambiante pendant une nuit. Le magnésien ainsi obtenu a été ajouté sous azote à une solution de 30,4 g (0,2 Mole) d'oxophorone dans 70 ml d'éther anhydre. L'addition a lieu en 45 min. pendant que la température s'élève de 25 à 35$^{o}$, puis le mélange a été maintenu sous agitation pendant une nuit à température ambiante. Après hydrolyse dans une solution aqueuse concentrée de chlorure d'ammonium, le mélange a été repris à l'éther, puis les parties éthérées ont été lavées, séchées et concentrées pour fournir 52 g d'un produit qui, par distillation à l'aide d'un four à boules à 13,3 Pa, fournit 48,9 g (rendement 84%) de l'hydroxy cétone désirée.

IR : 3450 et 1670 $cm^{-1}$ ;

RMN : 1,13 et 1,23 (6H, 2s) ; 1,25 (9H, s) ; 1,41 (3H, d, $J \simeq 6$ Hz) ; 2,12 (3H, s) ; 2,47 (2H, s) ; 4,17 à 4,52 (1H, m) ; 5,84 (1H, s) $\delta$ ppm ;

SM : $M^{+}$ = 278($\geq$0,1) ; m/e : 262(0,1), 222(3), 205(1), 191(11), 166(51), 148(100), 137(20), 124(27), 91(35), 77(28), 57(93), 41(76).

### Méthode B

On a préparé le magnésien conformément au procédé indiqué à la méthode A ci-dessus. Le mélange obtenu a été refroidi à 5$^{o}$, puis une solution de 30,4 g (0,2 Mole) d'oxophorone dans 70 ml d'éther y ont été ajoutés (temps d'introduction 20 minutes). Le mélange de réaction a été agité pendant 15 h à température ambiante, après quoi on a pu constater une disparition complète d'oxophorone de départ. Le produit désiré a été obtenu (47,3 g) en suivant le même procédé que celui indiqué à la méthode A (rendement 72,3%).

Le butynyl-tert-butyle éther, utilisé comme produit de départ dans le procédé décrit ci-dessus est un produit commercial ; il peut être obtenu à partir de 3-butyne-2-ol et isobutylène.

Exemple 2

2,6,6-Triméthyl-4-hydroxy-l-(2-tert-butyloxy-3,4-butadiène-4-yl)-2-cyclohexène

Méthode A (réduction à l'OMH)

On a procédé à la préparation du magnésien de 2,6,6-triméthyl-l-hydroxy-l-(3-tert- butoxy-l-butyne-l-yle)-2-cyclohexène-4-one comme indiqué à la méthode A de l'exemple 1. Le mélange a été ensuite réduit en ajoutant goutte à goutte 200 ml (0,4 Mole) de sodioaluminohydrure diéthylique (OMH) en mélange à 25% dans le toluène (temps d'introduction : 1 h 30). Le mélange a été maintenu sous agitation pendant une nuit, refroidi avec un bain d'eau glacée et hydrolysé en ajoutant avec précaution une solution d'acide acétique à 10% (300 ml), puis 200 ml d'HCl à 10%. Après dilution à l'éther, la phase organique a été séparée, lavée avec du NaOH 2N et avec de l'eau jusqu'à neutralité, séchée et concentrée. On a ainsi obtenu 44,5 g d'un produit brut.

Un échantillon analytique du produit désiré a été préparé par chromatographie en phase gazeuse.

IR : 3400, 1940 et 1070 $cm^{-1}$ ;

RMN : 1,1 et 1,17 (6H, 2s) ; 1,22 (9H, s) ; 1,23 (3H, d, J ≃ 6 Hz) ; 1,8 (3H, s) ; 2,0 (2H, s) ; 4,1-4,55 (2H, m) ; 5,5-5,97 (2H, m) δ ppm ;

SM : $M^+$ = 264( ⩾0,1) ; m/e : 246(1), 231 (0,1), 210(0,1), 194(0,1), 175(3), 162(7), 147(8), 131(7), 109(1), 101(17), 84(35), 68(10), 59(39), 57(100), 41(35).

Méthode B (réduction au $LiAlH_4$)

13,3 g (0,35 Mole) d'aluminohydrure de sodium ont été ajoutés par petites portions au mélange de magnésien préparé suivant la méthode A de l'Exemple 1. Après avoir été laissé sous agitation à température ambiante pendant une nuit, le mélange a été refroidi et hydrolysé avec précaution en ajoutant de l'eau, puis il a été dilué avec de l'HCl à 10% et repris avec de l'éther. La phase organique séparée a été lavée, séchée et concentrée pour fournir 41,1 g de produit brut. Le produit obtenu s'est montré identique à celui obtenu suivant la méthode A ci-dessus.

Exemple 3

β-Damascénone

Le mélange brut du composé allénique obtenu suivant l'Exemple 2 (4,45 g) a été chauffé à reflux sous atmosphère d'azote avec une solution de 250 ml d'acide formique à 50% et 250 ml de dioxanne. Le déroulement de la réaction a été suivi à l'aide de contrôle par gaz-chromatographie. Après 2 h 30, le mélange a été refroidi à l'aide d'un bain de glace, repris à l'éther et la phase organique une fois séparée a été lavée avec de l'eau, une solution 2N de NaOH et encore avec de l'eau jusqu'à neutralité. Après séchage et évaporation, on a obtenu 4,23 g d'un produit brut qui, par distillation, a fourni 2,09 g d'un produit ayant une teneur en β-damascénone de 68% environ (rendement 35% calculé sur la base de l'oxophorone de départ).

## REVENDICATIONS

1. Composés de formule

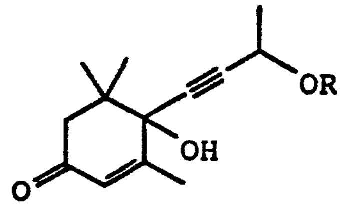

(I)

dans laquelle le symbole R représente un radical trialkyl-silyle, un radical alkyle $C_1$-$C_6$, de préférence méthyle, tert-butyle ou isoamyle ou un groupe de formule

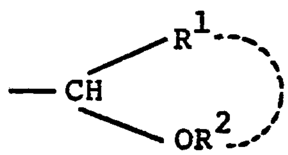

(II)

dans laquelle $R^1$ et $R^2$, pris séparément, représentent chacun un reste alkyle inférieur, de préférence de $C_1$ à $C_3$, ou, pris conjointement, un groupe tétraméthylène.

2. Procédé pour la préparation des composés de formule (I) suivant la revendication 1, caractérisé en ce qu'on fait réagir la 2,2,6-triméthyl-cyclohex-5-ène-1,4-dione ("oxophorone") avec un dérivé organo-métallique de formule

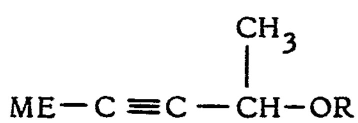

(III)

dans laquelle le symbole ME représente un radical halomagnésien et R un radical trialkyl-silyle, un radical alkyle, de préférence méthyle, tert-butyle ou isoamyle ou un groupe de formule (II) telle que définie à la revendication 1, et que l'on hydrolyse ensuite le produit de réaction ainsi obtenu.

3. Utilisation des composés selon la revendication 1 à titre de produits de départ pour la préparation de β-damascénone, caractérisée en ce qu'on réduit lesdits composés à l'aide d'un aluminohydrure et qu'on traite ensuite le produit résultant par un agent acide.

4. Utilisation suivant la revendication 3, caractérisée en ce que l'aluminohydrure est l'aluminohydrure de lithium, l'aluminohydrure diisobutylique (DiBAH) ou le sodioaluminohydrure diéthylique (OMH).

5. Composés de formule

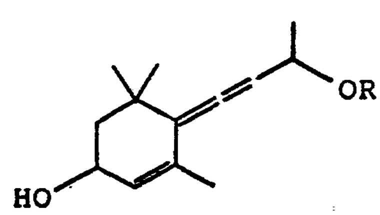

(IV)

dans laquelle le symbole R est défini comme indiqué à la revendication 1.

6. Procédé pour la préparation des composés de formule (IV) selon la revendication 5, caractérisé en ce qu'on fait réagir la 2,2,6-triméthyl-cyclohex-5-ène-1,4-dione ("oxophorone") avec un dérivé organo-métallique de formule

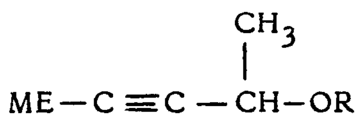

(III)

dans laquelle les symboles ME et R sont définis comme indiqué à la revendication 2, et réduit le produit d'addition résultant à l'aide d'un hydrure métallique en présence d'un solvant organique inerte appartenant à la classe des hydrocarbures aromatiques ou des éthers.

7. Procédé suivant la revendication 6, caractérisé en ce que l'hydrure métallique est le sodium alumino-hydrure diéthylique.

8. Utilisation des composés selon la revendication 5 à titre de produits de départ pour la préparation de β-damascénone, caractérisée en ce qu'on traite lesdits composés à l'aide d'un agent acide.

9. Utilisation suivant la revendication 7, caractérisée en ce que l'agent acide est un acide fort dilué, minéral ou organique.

10. Utilisation suivant la revendication 8, caractérisée en ce que l'acide fort est l'acide formique.